# EUROPÄISCHE PATENTSCHRIFT

(11) **EP 1 021 730 B1**
(45) Veröffentlichungstag und Bekanntmachung des Hinweises auf die Patenterteilung: **02.04.2003**
(21) Anmeldenummer: 98958198.8
(22) Anmeldetag: 13.10.1998
(51) Int. Cl.: G01R 33/28, A61B 5/055

(54) **MR-Bildgebungssystem und Vena-Cava-Filter zur Verwendung in diesem System**
MR imaging system and vena cava filter therefor
Système d'imagerie par résonance magnétique et filtre à veine cave pour ce système

(30) Priorität: 13.10.1997 DE 19746735
(43) Veröffentlichungstag der Anmeldung: 26.07.2000
(60) Teilanmeldung: 00250427.2
(73) Patentinhaber: Simag GmbH, 12247 Berlin (DE)
(72) Erfinder: Melzer, Andreas, 45478 Mülheim/Ruhr (DE); Busch, Martin, Dr., 58455 Witten (DE)
(74) Vertreter: Müller, Wolfram Hubertus, Dipl.-Phys.
(86) Internationale Anmeldenummer: DE9803046
(87) Internationale Veröffentlichungsnummer: WO99019739

(56) Entgegenhaltungen:
- EP-A- 0 385 367
- EP-A- 0 597 546
- EP-A- 0 602 970
- EP-A- 0 673 621
- EP-A- 0 768 539
- EP-A- 0 775 500
- WO-A-96/38083
- US-A- 4 960 106
- BURL M. ET AL.: "Tuned Fiducial Markers to Identify Body Locations with Minimal Perturbations of Tissue Magnetization", MAGNETIC RESONANCE IN MEDICINE, USA, 01. September 1996, Band 36, Nr. 3, Seiten 491 - 493
- FUKUSHIMA E. ET AL.: "Experimental Pulse NMR", 1981, ADDISON-WESLEY, MASSACHUSETTS

## Beschreibung

Die Erfindung betrifft ein MR (Magnetresonanz)-Bildgebungssystem nach dem Oberbegriff des Anspruchs 1 und einen auffaltbaren Vena-Cava-Filter zur Verwendung in einem derartigen MR-Bildgebungssystem.

### Hintergrund der Erfindung

MR-Bildgebungsverfahren sind seit längerem bekannt. Sie beruhen auf der Resonanz-Wechselwirkung zwischen einem hochfrequenten elektromagnetischen Wechselfeld und bestimmten Atomkernen eines zu untersuchenden Objektes, insbesondere eines menschlichen oder tierischen Körpers, das in einem starken äußeren Magnetfeld angeordnet ist. Die Atomkerne präzedieren im Magnetfeld (B₀) mit der sogenannten Lamorfrequenz, die proportional zur Stärke des Magnetfeldes ist. Bei Einstrahlen eines elektromagnetischen Wechselfelds, dessen magnetische Wechselkomponente (B₁) senkrecht zur Richtung des starken Magnetfelds (B₀) ist, werden die Spins der Atomkerne zum Umklappen gebracht und können damit zusammenhängende Relaxationszeiten gemessen werden.

Für die Beschreibung in einem wissenschaftlichen Modell wird die Magnetisierung der einzelnen Spins zu einer Gesamtmagnetisierung zusammengefaßt. Diese Gesamtmagnetisierung ist in ihrer Gleichgewichtslage parallel zum äußeren Magnetfeld und wird Gleichgewichtsmagnetisierung genannt. Durch einen mit der Lamorfrequenz (Resonanzfrequenz) eingestrahlten HF-Impuls kann die Magnetisierung um einen Winkel *α* in Bezug auf die Magnetfeldrichtung ausgelenkt werden. Der Winkel α ist proportional zur Dauer des eingestrahlten HF-Impulses und zur Magnetfeldstärke (B₁) des HF-Impulses. Nach einer Anregung um den Winkel α präzediert die Gesamtmagnetisierung um die Richtung des Magnetfeldes. Die präzedierende Magnetisierung kann mit einer Spule, die senkrecht zur Richtung des Magnetfeldes orientiert ist, als ein Spannungssignal aufgezeichnet werden. Die Stärke des Spannungssignals ist proportional zu sin(*α*), proportional zur Dichte der Spins im signalgebenden Volumen und umgekehrt proportional zur Temperatur.

Die maximale Signalantwort eines gegebenen Volumens wird daher nach einer 90° Anregung erreicht. Die aufgezeichnete Signalamplitude nimmt exponentiell mit der Relaxationszeit T₂* ab, da die einzelnen Spins auf Grund von fluktuierenden Magnetfeldern ihre Phasenbeziehung verlieren. Gleichzeitig nimmt die Gesamtmagnetisierung in Richtung des Magnetfeldes wieder exponentiell mit der Relaxationszeit T₁ auf die Gleichgewichtsmagnetisierung hin zu. Mit Hilfe von zu richtigen Zeitpunkten geschalteten magnetischen Gradientenfeldern ist es möglich, unterschiedliche Kombinationen aus der Spindichte und den beiden Relaxationszeiten ortsaufgelöst in einem grauwertkodierten Bild darzustellen.

Weiter ist es bekannt, mit Hilfe eines Schwingkreises lokal eine Verstärkung der Anregung von Kernspins herbeizuführen. Hierzu sind sogenannte "Fiducial Markers" bekannt, die spezielle, mit signalintensiven Flüssigkeiten gefüllte Kompartments aufweisen, die von einem Schwingkreis umgeben sind (Burl et al.: "Tuned Fiducial Markers to Identify Body Locations with Minimal Perturbation of Tissue Magnetization", in: Journal of Magnetic Resonance in Medicine 1996, 491-493). Der Schwingkreis besitzt dabei die Resonanzfrequenz des MR-Systems.

Wird ein solcher Fiducial Marker in das bildgebende Volumen eines Kernspintomographen eingebracht, so wird im Falle der Einstrahlung von elektromagnetischer Strahlung mit der Resonanzfrequenz der Schwingkreis angeregt. Dies führt zu einer Verstärkung des magnetischen Wechselfeldes innerhalb der Induktivität des Schwingkreises. Die erhöhte magnetische Wechselfeldkomponente vergrößert den Drehwinkel *α* der Protonen innerhalb der Induktivität. Bei einem kleinen Anregungswinkel (*α* < 90°) der Protonen durch das Kernspinsystem erfahren die Protonen innerhalb der Induktivität einen vergrößerten Anregungswinkel. Im Idealfall werden Protonen im bildgebenden Volumen mit einem kleinen Winkel von 1° - 10° angeregt, wogegen die Protonen innerhalb der Induktivtät mit 90° angeregt werden. Selbst bei identischen Relaxationszeiten und bei einer identischen Spindichte ist das Signal des vom Schwingkreis umgebenen Kompartments deutlich intensiver als das Signal der anderen Bildanteile. Da diese Signalanhebung lokalisiert ist, kann sie zur Positionsbestimmung verwandt werden.

Ebenso gilt nach dem Reziprozitätsgesetz, daß die MR-Antwortsignale der Protonen innerhalb des vom Schwingkreis umgebenden Kompartments (Fiducial Markers) verstärkt werden. Durch die Induktivität werden die Magnetfeldlinien, die von den Spins innerhalb der Spule herrühren, gebündelt, so daß mehr Signal aus dem Volumen innerhalb der Induktivität abgestrahlt und in eine zugeordneten Empfangsspule eingestrahlt wird. Diese Verstärkung der abgestrahlten und dann empfangenen Signale ist unabhängig von einer verstärkten Anregung zu sehen. Beide Effekte führen zu einer veränderten Signalantwort des Fiducial Markers.

Nachteilig verwenden "Fiducial Markers" gesonderte signalgebende Volumina, die zur Sichtbarkeit im MR-Bild eine Größe von zumindest einigen Kubikmillimetern aufweisen und im Untersuchungsobjekt extra plaziert oder in die Systeme integriert werden müssen, die im Untersuchungsobjekt plaziert werden. Dies ist häufig nicht möglich.

Mit Einführung offener Magnete und neuer Techniken bei geschlossenen MR-Systemen ist es inzwischen möglich, interventionelle und minimalinvasive Techniken wie Punktion, Katheterisierung und operative Verfahren unter MR-tomographischer Kontrolle durchzuführen. Dabei führen ferromagnetische oder paramagnetische Metalle oder Verunreinigungen anderer Werkstoffe zu Artefakten in der Bilddarstellung.

Bei den für interventionelle und minimalinvasive Techniken verwendeten Geräten ergeben sich Probleme, als sie meist aus einem ferromagnetischen oder paramagnetischen Material bestehen und/oder derart klein sind, daß sie in der Größenordnung der Pixelgröße (ca. 1mm) der MR-Bilder liegen. Insbesondere Katheter und Implantate aus Metall oder Kunststoffen sind im MR-Bild häufig nicht sichtbar und höchstens durch Artefakte lokalisierbar. Sofern im MR-Bild nicht sichtbare Stoffe verwendet werden, sind diese nur als "Schatten" erkennbar. Diese Nachteile führen dazu, daß eine MR-Überwachung interventioneller und minimalinvasiver Techniken häufig unbefriedigend ist und stattdessen ein röntgenologisches Verfahren zur Bilddarstellung verwendet wird, mit den bekannten Nachteilen derartiger Verfahren.

Aus der DE 195 10 194 A1 ist ein aktiv-invasives Magnetresonanzsystem zur Erzeugung selektiver MR-Angiogramme bekannt, bei dem ein Invasiv-Gerät mit einer HF-Spule versehen ist, mit deren Hilfe lokal die Kernspin-Magnetisierung des in dem Gefäß fließenden Blutes verändert wird. Mit speziellen MR-Bildimpulssequenzen wird selektiv nur das Blut erfaßt und dargestellt, das eine veränderte Kernspin-Magnetisierung aufweist.

Die US-A 5,445,151 beschreibt ein Verfahren zur Flußmessung strömender Flüssigkeiten, insbesondere von Blut, bei dem an einem Invasiv-Gerät mindestens zwei HF-Spulen vergesehen sind, wobei eine von der einen HF-Spule erzeugte lokale Veränderung der Kernspin-Magnetisierung an der anderen HF-Spule erfaßt und die Verzögerungszeit zur Bestimmung der Flußgeschwindigkeit ausgewertet wird.

Die beiden vorgenannten Druckschriften betreffen nicht die Darstellung in einen Körper eingebrachter medizinischer Vorrichtungen. Des weiteren haben sie den Nachteil, daß sie aktive Systeme darstellen, bei denen die eingeführten Geräte ständig über Kabelverbindungen mit extrakorporalen Komponenten in Verbindung stehen.

Die DE 195 07 617 A1 beschreibt ein MR-Verfahren, bei dem in ein Untersuchungsobjekt ein chirurgisches Instrument, etwa ein Katheter, eingeführt wird, an dessen Spitze eine Mikrospule befestigt ist. Die Position der Mikrospule wird unter Verwendung bestimmter Sequenztechniken bestimmt.

Aus der EP-A-0 768 539 ist ein MR-Verfahren zur Bestimmung der Position eines Objektes bekannt, das in den Körper eines Patienten eingebracht wird. Dabei wird eine Spulenanordnung ohne Verbindung mit extrakorporalen Komponenten an dem in den Körper einzuführenden Objekt, etwa einem Kathether oder einem chirurgischen Instrument, befestigt und eine aufgrund der Spule erfolgte Signaländerung zur Lokalisation des Objektes verwendet.

Die EP-A-0 385 367 beschreibt einen Ballonkatheter für vaginale und rektale Untersuchungen mit einem Schwingkreis mit einer Induktivität und einer Kapazität. Die Induktivität ist derart in den Ballon integriert, daß sie sich beim Ausdehnen des Ballons mit ausdehnt. Der Schwingkreis ist dabei über eine Kabelzuführung mit extrakorporalen Komponenten verbunden. Das Problem der Sichtbarmachung des Katheters im MR-Bild ist nicht angesprochen.

Ein ähnlicher Ballonkatheter, bei dem eine Induktivität in den Ballon des Katheter integriert ist, ist in der EP-A-0 597 546 beschrieben.

### Aufgabe der Erfindung

Der Erfindung liegt die Aufgabe zu Grunde, ein MR-Bildgebungssystem zur Darstellung und Positionsbestimmung einer in ein Untersuchungsobjekt eingeführten auffaltbaren medizinischen Vorrichtung sowie einen auffaltbaren Vena-Cava-Filter zur Verwendung in einem solchen MR-Bildgebungssystem zur Verfügung zu stellen, die eine deutliche und signalintensive Darstellung der Vorrichtung im MR-Bild ermöglichen.

### Zusammenfassung der Erfindung

Diese Aufgabe wird erfindungsgemäß durch ein MR-Bildgebungssystem mit den Merkmalen des Anspruchs 1 und einen auffaltbaren Vena-Cava-Filter mit den Merkmalen des Anspruchs 6 gelöst. Vorteilhafte und bevorzugte Ausgestaltungen der Erfindung sind in den Unteransprüchen angegeben.

Die erfindungsgemäße Lösung sieht vor, daß in einem lokal begrenzten Bereich des Untersuchungsobjekts innerhalb und/oder außerhalb einer medizinischen Vorrichtung eine veränderte Signalantwort erzeugt wird. Dazu wird ein geschlossener Schwingkreises mit einer Induktivität und einer Kapazität verwendet, dessen Resonanzfrequenz im wesentlichen gleich der Resonanzfrequenz der eingestrahlten hochfrequenten Strahlung ist. Erfindungsgemäß bildet ein auffaltbarer Teil der Vorrichtung die Induktivität aus oder integriert der auffaltbare Teil diese in sich, so daß sie sich beim Auffalten der Vorrichtung im Untersuchungsobjekt zusammen mit dieser auffaltet.

Dadurch wird ein MR-Bildgebungssystem mit einer Vorrichtung zur Verfügung gestellt, die eine verbesserte Darstellung im MR-Bild auch bei Vorliegen auffaltbarer Teile der Vorrichtung gewährleistet. Darüberhinaus entsteht aufgrund der Ausbildung der Induktivität durch den auffaltbaren Teil bzw. die Integration der Induktivität in den auffaltbaren Teil ein synergistischer Effekt.

Über den im MR-Bild entsprechend hervorgehobenen Bereich ist die Position der Vorrichtung deutlich feststellbar, da dieser Bereich unmittelbar von innen oder außen- an die Vorrichtung angrenzt. Da eine veränderte Signalantwort des zu untersuchenden Objekts selbst erzeugt wird, treten dabei nur solche Artefakte auf, wie sie durch das Material der Vorrichtung erzeugt werden.

Durch die deutliche Darstellung der Vorrichtung im MR-Bild ist eine genaue Positionsbestimmung möglich. Aufgrund der veränderten Signalverhältnisse ist darüber hinaus auch eine verbesserte Flußmessung im Falle eines durch die Vorrichtung oder an der Vorrichtung vorbei strömenden Fluids möglich. Dabei wird ausgenutzt, daß innerhalb und außerhalb der Vorrichtung eine unterschiedliche Anregung vorliegt.

Die Signalantwort der Spins innerhalb der Induktivität wird erfindungsgemäß verändert. Hierzu tragen zwei Vorgänge bei. Zum einen wird bei Einstrahlung der hochfrequenten Strahlung der auf die Resonanzfrequenz abgestimmte Schwingkreis angeregt und erfahren die vom Feld des Schwingkreises erfaßten Kernspins eine verstärkte Anregung durch eine lokale Verstärkung des wechselnden magnetischen Feldes in oder in der Nähe der Induktivität. Mit anderen Worten werden von den Feldlinien des induzierten Magnetfeldes erfaßte Protonen um einen größeren Winkel gedreht als Protonen außerhalb dieses induzierten Magnetfeldes. Es erfolgt ein verstärktes Umkippen der Kernspins. Entsprechend kann die von einer Empfangsspule erfaßte und zur Bilddarstellung ausgewertete Signalantwort verstärkt sein. Ebenfalls ist möglich, daß nur die Spins innerhalb der Induktivität eine Sättigung erfahren und das Signal gegenüber der Umgebung abgeschwächt ist. In beiden Fällen liegt eine Änderung der Signalantwort vor.

Zum anderen werden - unabhängig von einer verstärkten Anregung - die MR-Antwortsignale der Protonen innerhalb der Induktivität verstärkt. So werden durch die Induktivität die Magnetfeldlinien, die von den Spins innerhalb der Induktivität herrühren, gebündelt, so daß mehr Signal abgestrahlt und in eine zugeordneten Empfangsspule eingestrahlt wird, die die verstärkten Signale empfängt und zur MR-Bildgebung weiterleitet. Dieser Effekt ist in der Veröffentlichung J. Tanttu: "Floating Surface Coils", in: XIV ICMBE AND VII ICMP, Espoo, Finland 1985" beschrieben.

Diese beiden Effekte können bei dem erfindungsgemäßen MR-Bildgebungssystem beide zur Veränderung der Signalantwort genutzt werden. Es kann jedoch auch allein der zweite Effekt, d.h. eine Verstärkung der MR-Antwortsignale, ausgewertet werden.

Dementsprechend ist bei einer ersten Ausgestaltung der Erfindung vorgesehen, daß bei Einstrahlung der hochfrequenten Strahlung der Schwingkreis angeregt und dadurch in dem lokal begrenzten Bereich eine verstärkte Anregung der Kernspins des Untersuchungsobjekts erfolgt.

Der lokal begrenzte Bereich, in dem eine Verstärkung der Anregung der Kernspins erfolgt, kann zum einen in einem innerhalb der Vorrichtung ausgebildeten Kompartment liegen, das von der Induktivität umgeben ist. Es wird also ein im Inneren der Induktivität bzw. Spule angeordnetes Volumen des Untersuchungsobjektes verstärkt dargestellt.

Zum anderen kann dieser Bereich außerhalb der Vorrichtung liegen und an diese angrenzen, wobei mindestens ein Schwingkreis derart an der Oberfläche der Vorrichtung angeordnet ist, daß bei Einstrahlung hochfrequenter Strahlung eine Verstärkung des magnetischen Flusses in dem betrachteten angrenzenden Bereich erfolgt.

Eine zweite Ausgestaltung der Erfindung sieht vor, daß bei Einstrahlung der hochfrequenten Strahlung der Schwingkreis verstimmt oder die Kapazität kurzgeschlossen wird, so daß keine verstärkte Anregung der Kernspins in dem lokal begrenzten Bereich erfolgt. Bei Messung der Signalantwort des lokal begrenzten Bereichs wird die Verstimmung des Schwingkreises bzw. der Kurzschluß der Kapazität jedoch wieder aufgehoben, so daß der Schwingkreis eine Verstärkung der abgestrahlten MR-Antwortsignale der Protonen bewirkt. Es hat sich gezeigt, daß insbesondere diese Variante es ermöglicht, mit hoher Qualität den Bereich in und um die Vorrichtung abzubilden, d.h. über eine reine Positionsbestimmung hinaus eine lokale Bildgebung zur Verfügung stellt. Neben der Position der Vorrichtung sind dem MR-Bild verbesserte Aussagen über die Gewebestruktur etc. des Inneren und/oder der Umgebung der Vorrichtung zu entnehmen.

Eine Verstärkung der Anregung der Kernspins wird beispielsweise dadurch unterdrückt, daß der Kondensator des Schwingkreises durch gekreuzte Dioden bei der Anregung kurzgeschlossen wird. Die Verstärkung der abgestrahlten Signale wird dadurch nicht beeinflußt, da die geringe induzierte Spannung durch die innerhalb der Induktivität befindlichen Spins bei der Abstrahlung unterhalb der Durchlaßspannung der Dioden liegt.

Es wird allgemein darauf hingewiesen, daß die erfindungsgemäße Änderung der Signalantwort meist eine Verstärkung der Signalantwort sein wird. Dies hängt jedoch von zahlreichen Faktoren ab, insbesondere den verwendeten Anregungssequenzen. Beispielsweise kann bei schnell aufeinanderfolgenden Sequenzen eine Sättigung der Anregung der Spins innerhalb der Induktivität vorliegen, so daß dort kein Signal erzeugt wird. In dem Bereich außerhalb der Induktivität, in dem eine geringere Anregung der Kernspins erfolgt, liegt dagegen keine Sättigung vor, so daß hier ein Signal erzeugt wird. Entsprechend erfolgt bei diesem Beispiel eine Verminderung der Signalanwort im vom Feld der Induktivität erfaßten Bereich.

Mit Vorteil ist vorgesehen, daß zur resonanten Abstimmung des Schwingkreises die Induktivität und/oder die Kapazität eingestellt werden können. Dies ist für die Fälle sinnvoll, bei denen nach Einbringen in das Untersuchungsobjekt und einer möglichen Aufweitung der Vorrichtung oder von Teilen der Vorrichtung sich das Produkt von Induktivität und Kapazität und somit die Resonanzfrequenz des Schwingkreises ändern.

Der erfindungsgemässe auffaltbare Vena-Cava-Filter zeichnet sich durch mindestens einen geschlossenen Schwingkreis mit einer Induktivität und einer Kapazität aus, wobei die Induktivität in einen auffaltbaren Teil des Vena-Cava-Filters derart integriert ist, daß sie sich bei Auffalten des Vena-Cava-Filters zusammen mit diesem auffaltet.

Der Vena-Cava-Pilter weist bevorzugt längliche, beweglichen Verzahnungselementen auf, wobei die Induktivität an den Verzahnungselementen befestigt ist, so dass sie sich beim Auffalten der Verzahnungselemente zusammen mit diesen auffaltet.

Es kann vorgesehen sein, dass die Induktität und/oder die Kapazität aus dem Material des Vena Cava Filters gebildet ist.

### Beschreibung mehrerer Ausführungsbeispiele

Die Erfindung wird nachfolgend unter Bezugnahme auf die Figuren der Zeichnung an mehreren Ausführungsbeispielen näher erläutert. Es zeigen:
- Fig. 1a, b -: schematisch zwei Ausführungsbeispiele eines Kathethers bzw. Führungsdrahts;
- Fig. 2a-2g -: verschiedene elektrische Schaltbilder eines Schwingkreises;
- Fig. 3a, 3b -: zwei Ausführungsbeispiele eines Ballonkathethers;
- Fig. 4a, 4b -: zwei Ausführungsbeispiele eines Vena Cava Filters und
- Fig. 5a, 5b -: zwei Ausführungsbeispiele einer Herzklappe.

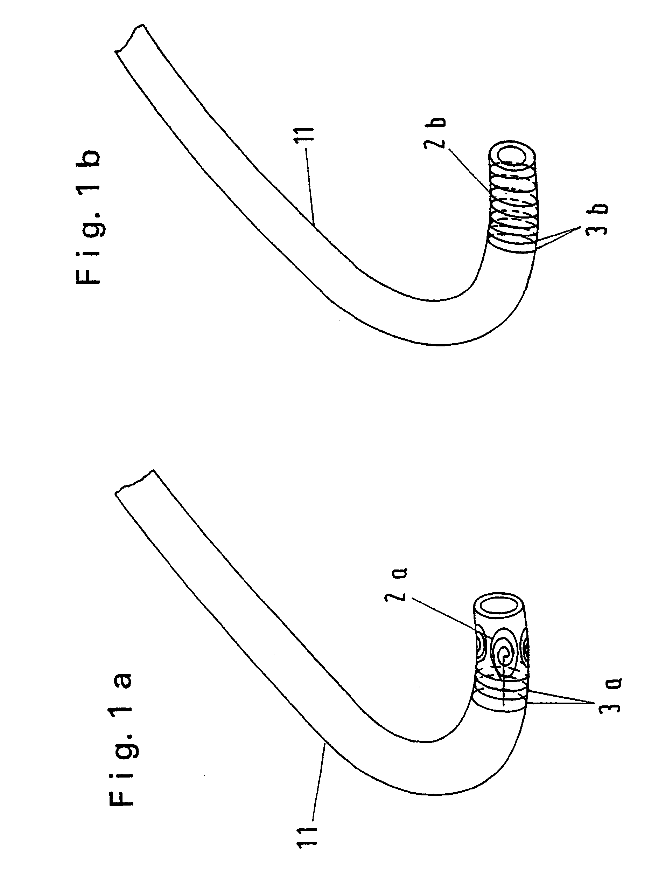

Figuren 1a und 1b zeigen einen Führungsdraht oder Kathether 11, an dessen Spitze jeweils ein Schwingkreis bestehend aus einer Induktivität 2a, 2b und einem Kondensator 3a, 3b ausgebildet ist. In Fig. 1a wird die Induktivität durch spiralförmigen Leiter 2a ausgebildet (Solenoidspule), so daß das induzierte Magnetfeld im wesentlichen in senkrechter Richtung von dem Katherer 11 in das umgebende Gewebe gerichtet ist und dort eine verstärkte Anregung der Kernspins bewirkt. In Fig. 1b wird die Induktivität durch eine helixförmige Spule 2b ausgebildet, so daß das induzierte Magnetfeld im wesentlichen parallel zur Längsachse des Katheters 11 verläuft und im Inneren des Katheters 11 eine verstärkte Anregung der Kernspins bewirkt. Der Kondensator 3a, 3b ist jeweils durch parallele, ringförmige Leiterelemente verwirklicht. Alternativ kann der Kondensator auch durch ein gesondertes Bauelement, das in den Katheter 11 integriert ist, verwirklicht werden.

Die Induktivität 2a, 2b und Kapazität 3a, 3b sind bevorzugt auf einer Folie ausgebildet, etwa mittels eines photolitographischen Verfahrens. Die Folie wird auf einen flexiblen Schlauch (nicht gesondert dargestellt) aufgebracht. Nach einer Vesiegelung von Schlauch und Folie wird der Schlauch auf den Führungsdraht oder Katheter 11 aufgebracht, so daß sich die dargestellte Anordnung ergibt.

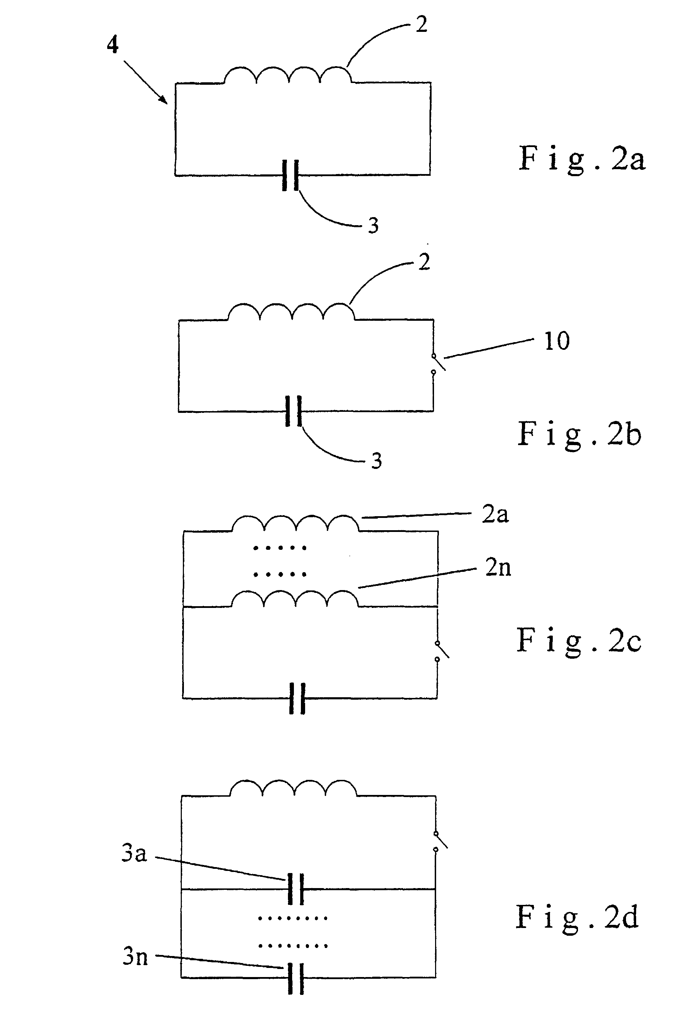

In Figur 2a ist das elektrische Schaltbild des im Katheter 11 ausgebildeten Schwingkreises 4, bestehend aus Induktivität 2 und Kapazität 3, dargestellt. Optional ist gemäß Fig. 2b zusätzlich ein Schalter 10 vorgesehen, der mechanisch, etwa durch einen Betätigungsdraht des Katheters 11, elektrisch oder magnetisch aktivierbar bzw. deaktivierbar ist.

Der Schwingkreis 4 kann auf vielfältigste Weise ausgebildet sein. Gemäß Fig. 2c kann er mehrere parallel geschaltete Induktivitäten 2a bis 2n und gemäß Fig. 2d mehrere parallel geschaltete Kapazitäten 3a bis 3n aufweisen. Des weiteren können mehrere Induktivitäten und/oder Kapazitäten seriell geschaltet sein. Auch kann vorgesehen sein, an einer Vorrichtung mehrere Schwingkreise auszubilden, die jeweils einen Schalter und seriell und/oder parallel geschaltete Induktivitäten und/oder Kapazitäten aufweisen können.

Der Schwingkreis 4 weist eine Resonanzfrequenz auf, die der Resonanzfrequenz der eingestrahlten hochfrequenten Strahlung des MR-Bildungsgebungssystems entspricht, in dem der menschliche Körper, in den der Katheter appliziert wird, angeordnet ist.

Bei dem Katheter 1 wird der Schwingkreis 4 durch die eingestrahlten hochfrequenten Pulse des MR-Systems angeregt, da seine Resonanzfrequenz der Frequenz der eingestrahlten HF-Pulse entspricht. Dies führt zu einer Verstärkung des Magnetfeldes in der Induktivität des Schwingkreises oder in der Nähe der Induktivität, was wiederum zu einer verstärkten Anregung der Protonen im entsprechenden Bereich führen kann. Bei einer Anregung der Kerne außerhalb des vom Magnetfeld der Induktivität erfaßten Bereichs um einen Winkel, der kleiner als 90° ist, können Kerne im vom Magnetfeld der Induktivität erfaßten Bereich eine Anregung von 90° erfahren und so mit maximaler Amplitude antworten. Die im Bereich der Induktivität angeordneten Protonen bzw. Kerne erfahren somit eine stärkere Anregung, als außerhalb der Induktivität angeordneten Protonen.

Die Verstärkung des Drehwinkels innerhalb der Induktivität bzw. im vom Magnetfeld der Induktivität erfaßten Bereich kann gegenüber den Protonen außerhalb der Induktivität einen Faktor von bis zu 45 betragen. Es ist daher möglich, die Protonen im Inneren der Induktivität um einen Winkel von 90° auszulenken (maximale Signalantwort), während die Protonen außerhalb der Induktivität bzw. außerhalb des durch den Schwingkreis erzeugten magnetischen Feldes lediglich eine Kleinwinkelanregung von ca. 2° bis 10° erfahren. Dies führt dazu, daß im Fall der Fig. 1b der innere Bereich des Katheters 11 und im Falle der Fig. 1a der an den Katheter 11 angrenzende Bereich in einem MR-Bild wesentlich heller dargestellt wird als die restliche Umgebung. Es kann daher die Lokalisation des Katheters 11 im menschlichen Körper genau bestimmt werden.

Es folgt zur weiteren Veranschaulichung eine Abschätzung der erforderlichen Kapazitäten und Induktivitäten. In dem Beispiel wird ein Plattenkondensator verwendet und die Spule wird als Helix mit einer festen Windungszahl angenommen. Die Resonanzfrequenz eines Kernspinsystems liegt üblicherweise im Bereich zwischen 2 MHz bis 90 MHz. Die Resonanzfrequenz des Kernspinsystems ist dabei gleich dem Produkt aus der magnetischen Feldstärke und dem gyromagnetischen Verhältnis g. Bei einer mittleren Feldstärke von 1 Tesla ergibt sich eine Resonanzfrequenz von ca. 42 MHz. Die Resonanzfrequenz des Schwingkreises ergibt sich aus der Thomsonschen Schwingungsgleichung. Sie ist umgekehrt proportional zur Wurzel aus dem Produkt der Induktivität und der Kapazität.

Das Produkt aus Kapazität und Induktivität ist dann gleich 1,4 x 10⁻¹⁹ S². Bei einem angenommenen Durchmesser des Kathethers 11 der Figur 1b von 8 mm und einer Länge der Spule 2b von 40 mm ergibt sich je nach Windungszahl eine Induktivität von etwa 4 x 10⁻⁶ Vs/A. Die sich daraus ergebende resultierende Fläche eines Plattenkondensators bei einer relativen Dielektrizitätskonstanten von 2 und einem Abstand von 0,1 mm der einzelnen Platten des Plattenkondensators beträgt etwa 0,2 mm². Eine derart kleine Fläche eines Plattenkondensators ist in einem Katheter leicht zu verwirklichen. Bei höheren Magnetfeldern bzw. Frequenzen läßt sich die resultierende Fläche eines Plattenkondensators auf 0,014 mm² weiter reduzieren.

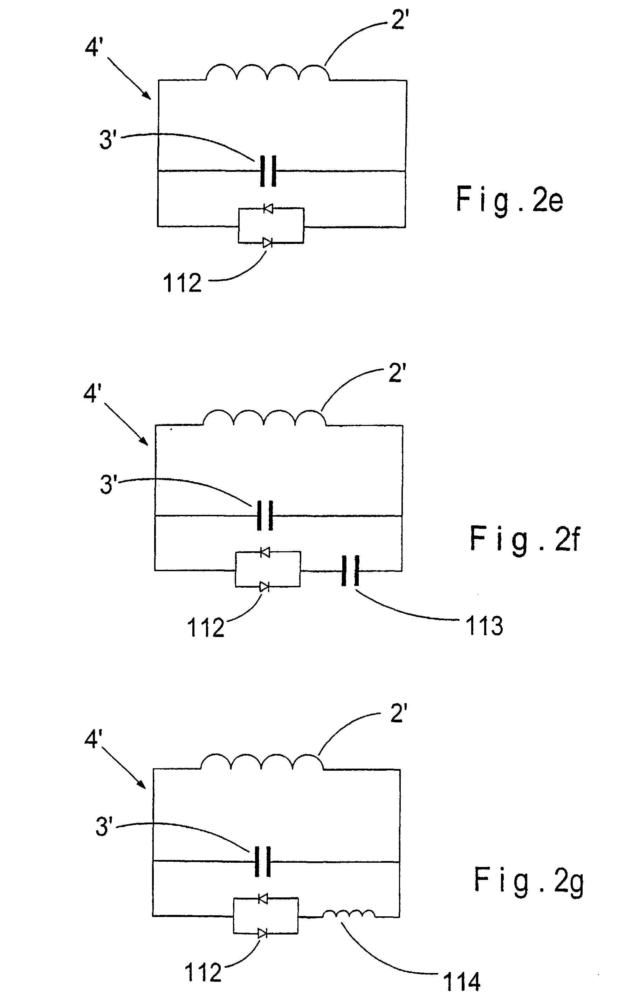

Zwei weitere Varianten des Schwingkreises sind anhand der Schaltbilder der Figuren 2e bis 2g dargestellt. In Figur 2e wird der Kondensator 3' durch zwei gekreuzte Dioden 112, die als zusätzliche Elemente im Katheter enthalten sind, während der Anregungsphase kurzgeschlossen. Die Dioden 112 weisen eine Durchlaßspannung auf, die etwa bei 1 Volt, jedenfalls unterhalb der bei der Einstrahlung hochfrequenter Strahlung erzeugten Spannung liegt, die üblicherweise mehr als 1 Volt beträgt. Die Dioden 112 leiten somit bei Einstrahlung der hochfrequenten Strahlung, so daß der Kondensator 3' in der Anregungsphase kurzgeschlossen wird und sich kein Schwinkreis ausbildet.

Dies bedeutet, daß bei Einstrahlung der hochfrequenten Strahlung anders als bei den bisherigen Ausgestaltungen keine verstärkte lokale Anregung der Kernspins erfolgt. Allerdings wird bei Messung der Signalantwort des von der Induktivität 2' erfaßten Bereiches der Kurzschluß der Kapazität 3' wieder aufgehoben. Hierzu sind die Dioden 112 derart ausgestaltet, daß die Durchlaßspannung oberhalb der bei der Spin-Signalantwort erzeugten Spannung liegt. Der Kondensator 3' wird somit bei Abstrahlung der MR-Antwortsignale der Atomkerne nicht kurzgeschlossen und es entsteht ein Schwingkreis 4', der eine Verstärkung der abgestrahlten MR-Antwortsignale der Protonen bewirkt und dadurch die gemessene Signalantwort verändert.

Die Dioden 112 können auf vielfältige Art im Katheter verwirklicht werden. Insbesondere können gesonderte Bauelemente verwendet oder die Dioden durch oder in Zusammenwirkung mit dem Material des Katheters gebildet werden, etwa als auf den Katheter aufgebrachte Struktur.

In Fig. 2f wird der Kondensator 3' bei prinzipiell gleichem Aufbau wie in Fig. 2e nicht kurzgeschlossen, sondern der Schwingkreis 4' durch Zuschalten eines weiteren Kondensators 113 in der Anregungsphase lediglich verstimmt, so daß eine verstärkte Anregung der Kernspins in nur begrenztem Maße erfolgt. Bei Abstrahlung der MR-Antwortsignale sperren die Dioden 112, so daß der Schwinkreis 4' dann unverstimmt vorliegt und eine Verstärkung der abgestrahlten MR-Antwortsignale erfolgt, was zu einer veränderten Signalantwort führt, die im MR-Bild dargestellt wird.

In Fig. 2g wird in der Anregungsphase der Schwingkreis 4''' nicht durch Zuschalten eines Kondensators, sondern durch Zuschalten einer Spule 114 verstimmt.

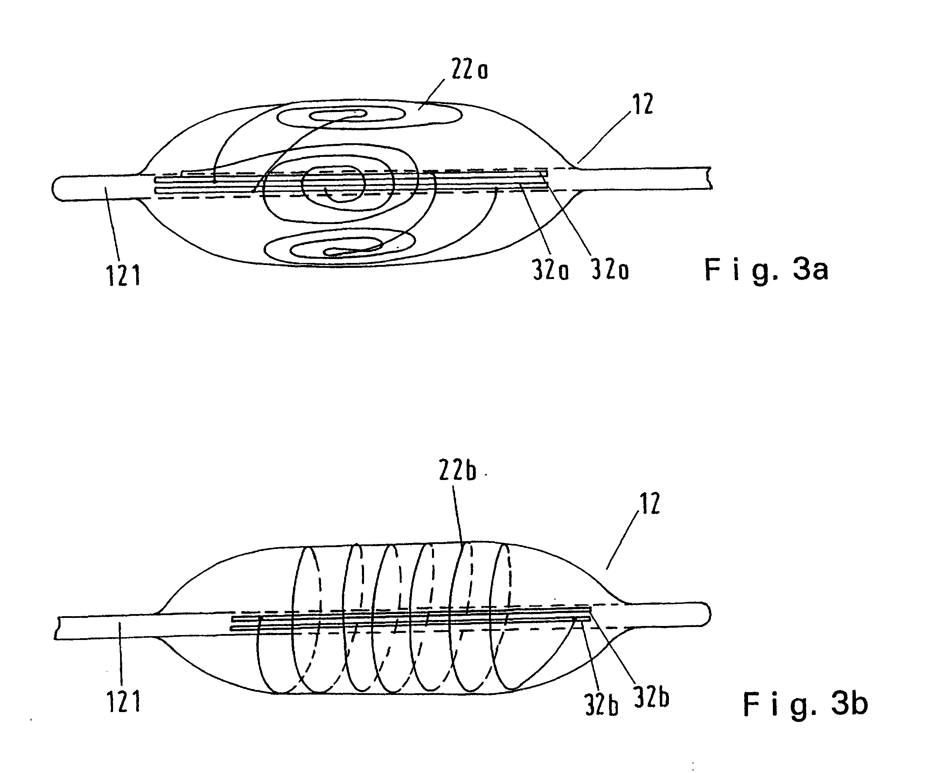

Es wird darauf hingewiesen, daß ein Kurzschluß oder eine Verstimmung des Schwingkreises in der Anregungsphase bei beliebigen, an einer medizinischen Vorrichtung ausgebildeten oder angeordneten Schwingkreisen verwirklicht werden kann, insbesondere auch an den nachfolgend beschriebenen Vorrichtungen der Fig. 3a bis 5b.

Figuren 3a, 3b zeigen jeweils einen Ballonkatheter 12 mit Schwingkreis. In Fig. 3a sind auf die Außenhaut des Ballonkatheters mehrere spiralförmige Induktivitäten 22a aufgebracht, deren Achse senkrecht zur Längsachse 121 des Ballonkatheters 12 verläuft. In Fig. 3b ist eine helixförmige Induktivität 22b vorgesehen, deren Achse parallel zur Längsachse 121 des Ballonkatheters 12 verläuft. In Kapazität 32a, 32b ist jeweils auf der Achse 121 des Ballonkatheters 12 in Form von parallelen Leitern verwirklicht. Die Induktivität 22a, 22b ist beispielsweise, wie in Bezug auf Fig. 1a, 1b beschrieben, auf einer Folie ausgebildet.

Zur Abstimmung der Resonanzfrequenz des Schwingkreises auf die Frequenz der eingestrahlten HF-Pulse sind verschiedene Gestaltungen des Schwingkreises möglich.

In einer Variante ist vorgesehen, daß die Güte des Schwingkreises relativ gering gehalten wird, um einen möglichst breitbandigen Schwingkreis zu realisieren und einen möglichst großen Bereich von Resonanzfrequenzen abzudecken.

Eine zweite Variante sieht vor, die Vorrichtung so auszubilden, daß auch nach einer Änderung der Geometrie, im betrachteten Beispiel nach Aufblasen des Ballonkatheters 12, das Produkt aus Induktivität und Kapazität konstant ist. Dies kann entweder dadurch erfolgen, daß dem Ballonkatheter eine Geometrie gegeben wird, die bei einer Entfaltung des Ballonkatheters ihre Eigenschaften möglichst wenig ändert, also insbesondere eine konstante Induktivität und eine konstante Kapazität aufweist. Ein Aufblasen des Ballonkatheters am Ort der Applikation bewirkt somit im wesentlichen keine Änderung der Resonanzfrequenz des Schwingkreises.

Eine Konstanz des Produktes von Induktivität und Kapazität kann zum anderen durch eine Kompensierung der sich ändernden Induktivität durch eine sich entsprechend ändernde Kapazität verwirklicht werden. Zur Kompensierung einer sich ändernden Induktivität durch eine sich entsprechend ändernde Kapazität ist beispielsweise vorgesehen, daß die Kondensatorflächen senkrecht oder parallel zueinander verschiebbar angeordnet ist, wobei sich die Kapazität entsprechend dem Abstand der Kondensatorflächen vergrößert bzw. verkleinert. Etwa kann in Fig. 3b eine Längsverschiebbarkeit der beiden Kondensatorplatten 32b bei Aufblasen des Ballonkatheters vorgesehen sein, um die Änderung der Induktivität beim Aufblasen zu kompensieren.

Eine dritte Variante sieht vor, daß eine Anpassung des Schwingkreises im Magnetfeld des Kernspintomographen durch eine Veränderung bzw. Einstellung der Induktivität und/oder Kapazität des Schwingkreises nach deren Plazierung erfolgt. Hierzu ist beispielsweise vorgesehen, die Kondensatorfläche mit Hilfe des im Körper befindlichen Applikationsinstruments zu verändern. Eine Verminderung der Induktivität und damit eine Anpassung des Schwingkreises auf die Resonanzfrequenz im Kernspintomographen kann etwa durch eine laserinduzierte, mechanische oder elektrolytische Isolierung von Spulensegmenten erfolgen. Eine Änderung der Kapazität kann ebenso durch eine laserinduzierte, mechanische oder elektrolytische Isolierung von Kapazitäten erfolgen.

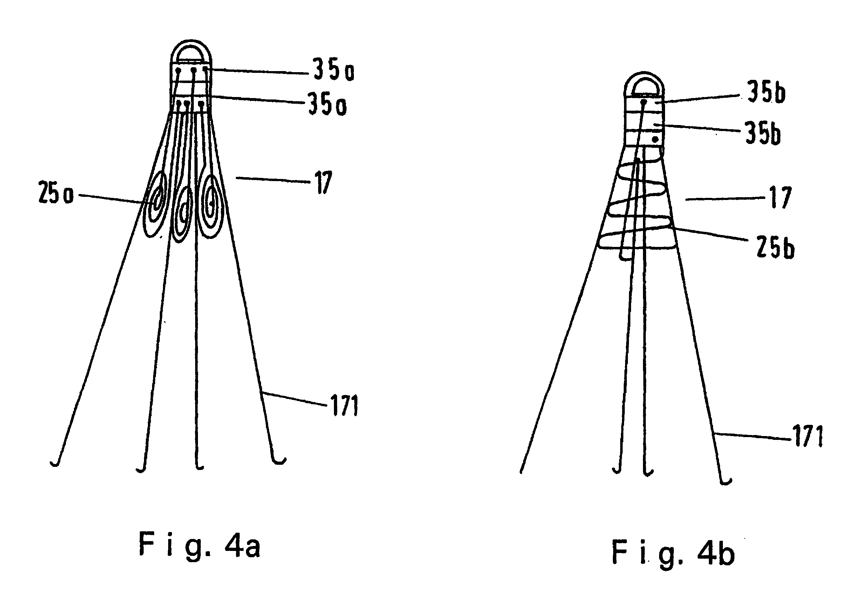

In den Fig. 4a, 4b ist ein Schwingkreis jeweils an einem Vena Cava Filter 17 ausgebildet. Ein Vena Cava Filter wird insbesondere zum Schutz vor Venentrombosen als eine Art Trichter in eine Vene eingesetzt. Über Verzahnungselemente 171 ist der Filter an der Gefäßwand befestigt. Die Induktivität 25a, 25b ist wiederum spiralförmig (Fig. 4a) oder helixförmig (Fig. 4b) ausgebildet. Die Kapazität 35a, 35b wird beispielsweise wiederum durch parallele, ringförmige kapazitive Elemente ausgebildet.

Die Induktivitäten 25a, 25b werden bevorzugt mit einem Laser aus Blech geschnitten. Sie sind in geeigneter Weise an den Verzahnungselementen 171 befestigt und dienen zusätzlich zur Stabilisierung.

Es ist ebenfalls möglich, die Induktivitäten 25a, 25b und ggf. auch die Kapazitäten 35a, 35b aus dem Material des Vena Cava Filters 17 zu bilden. Dabei werden Filter und Induktivitäten/Kapazitäten etwa durch an sich bekannte Laser- oder Funkenerosion- oder Wasserstrahl-Schneidetechniken aus einem geeigneten leitenden Material geschnitten.

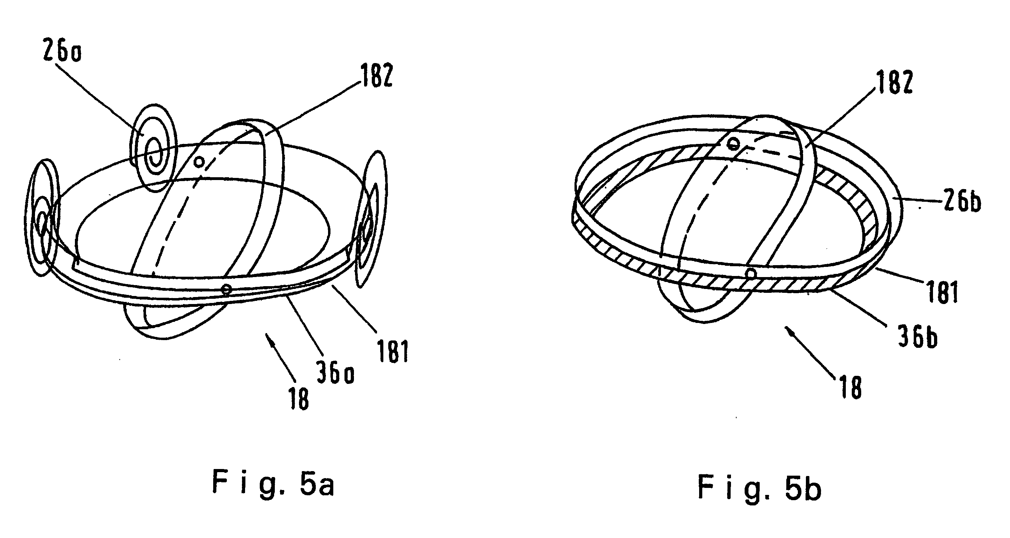

Die Figuren 5a, 5b schließlich zeigen eine Herzklappe 18 mit einem Ring 181, der in das Herzgewebe eingenäht wird und an dem beweglich die eigentliche Herzklappe 182 angeordnet ist. Zur Ausbildung eines Schwingkreises, der eine verstärkte Anregung der Kernspins bewirkt, ist zum einen in den Ring 181 ein Kondensator 36a, 36b integriert, etwa in Form paralleler ringförmiger Leiter. In Fig. 5a sind als Induktivität Solenoidspulen 26a vorgesehen, die sich am Umfang des Ringes entfalten. In Fig. 5b ist in den Ring 181 zusätzlich zum Kondensator 36b als Induktivität des Schwingkreises eine Ringspule 26b integriert.

Es wird darauf hingewiesen, daß zu den Ausführungsbeispielen der Figuren 1a, 1b bis 5a, 5b jeweils auch eine Kombination der verschiedenen Spulenanordnungen vorgesehen sein kann.

In einer Variante der Vorrichtung (nicht dargestellt) wird diese auch zur Flußmessung eingesetzt, sofern sie wie etwa der Vena Cava Filter der Fig. 4 von einem Fluid durchflossen oder umströmt wird. Die Vorrichtung weist dabei bevorzugt zwei hintereinander angeordnete Schwingkreise auf, wobei der eine Schwingkreis zwei gekreuzte Dioden entsprechend Figur 2e aufweist, so daß die Kapazität bei der Anregung kurzgeschlossen wird, während der zweite Schwingkreis ohne Dioden ausgebildet ist. Dies führt dazu, daß bei Einstrahlung hochfrequenter MR-Anregungsimpulse in einem Teilbereich der Vorrichtung, der von dem Schwingkreis ohne Dioden umgeben ist, eine verstärkte Anregung stattfindet. Jedoch liegt auch in dem anderen Teilbereich, der von dem Schwingkreis mit Dioden umgeben ist, eine gegenüber dem umgebenden Gewebe veränderte Signalantwort vor, wie anhand der Figur 2e erläutert wurde. Eine derartige Anordnung ist bei Verwendung geeigneter Sequenztechniken besonders gut zur Flußbestimmung und damit zur funktionellen Kontrolle der Vorrichtung geeignet.

Es wird darauf hingewiesen, dass die in den Figuren 1 und 5a, 5b dargestellten Vorrichtungen lediglich der Erläuterung des Hintergrunds der Erfindung dienen und diese nicht direkt betreffen.

## Patentansprüche

1. MR-Bildgebungssystem mit:
a) Mitteln zur Erzeugung eines äußeren Magnetfeldes um ein Untersuchungsobjekt,
b) Mitteln zur Einstrahlung hochfrequenter Strahlung einer bestimmten Resonanzfrequenz in das Untersuchungsobjekt, wobei Übergänge zwischen Spin-Energieniveaus der Atomkerne des Untersuchungsobjekts angeregt werden,
c) einer in das Untersuchungsobjekt einbringbaren medizinische Vorrichtung mit mindestens einem geschlossenen Schwingkreises mit einer Induktivität (22a, 22b, 25a, 25b) und einer Kapazität (32a, 32b, 35a, 35b), dessen Resonanzfrequenz im wesentlichen gleich der Resonanzfrequenz der eingestrahlten hochfrequenten Strahlung ist, und
d) mindestens einer Empfangsspule zur Detektion einer Signalantwort
**dadurch gekennzeichnet, daß**
ein auffaltbarer Teil der Vorrichtung die Induktivität (22a, 22b; 25a, 25b) ausbildet oder die Induktivität (22a, 22b, 25a, 25b) in einen solchen Teil derart integriert ist, daß sie sich beim Auffalten der Vorrichtung im Untersuchungsobjekt zusammen mit dieser auffaltet.

2. MR-Bildgebungssystem nach Anspruch 1, **dadurch gekennzeichnet, daß** die Vorrichtung Mittel (113) aufweist, welche mindestens einen Schwingkreis bei Einstrahlung der hochfrequenten Strahlung verstimmen.

3. MR-Bildgebungssystem nach Anspruch 1 oder 2, **dadurch gekennzeichnet, daß** die Vorrichtung Mittel (112) zum Kurzschließen der Kapazität (3') bei Einstrahlung der hochfrequenten Strahlung aufweist.

4. MR-Bildgebungssystem nach mindestens einem der Ansprüche 1 bis 13, **dadurch gekennzeichnet, daß** die Induktivität (2) und/oder die Kapazität (3) des Schwingkreises zur Abstimmung auf die Resonanzfrequenz des MR-Systems verstellbar sind.

5. MR-Bildgebungssystem nach mindestens einem der Ansprüche 1 bis 14, **dadurch gekennzeichnet, daß** der Schwingkreis (4) mehrere parallel oder seriell geschaltete Induktivitäten (2a, 2n) und/oder Kapazitäten (3a, 3n) aufweist.

6. Auffaltbarer Vena-Cava-Filter zur Verwendung in einem MR-Bildgebungssystem nach Anspruch 1,
**gekennzeichnet durch**
mindestens einen geschlossenen Schwingkreis mit einer Induktivität (25a, 25b) und einer Kapazität (35a, 35b), wobei die Induktivität (25a, 25b) in einen auffaltbaren Teil des Vena-Cava-Filters derart integriert ist, daß sie sich bei Auffalten des Vena-Cava-Filters zusammen mit diesem auffaltet.

7. Vena-Cava-Filter nach Anspruch 6, **dadurch gekennzeichnet, daß** die Induktivität (25a, 25b) spiralförmig oder helixförmig ausgebildet ist.

8. Vena-Cava-Filter nach Anspruch 6 oder 7, **dadurch gekennzeichnet, daß** die Kapazität (35a, 35b) durch parallele, ringförmige kapazitive Elemente ausgebildet ist.

9. Vena-Cava-Filter nach mindestens einem der Ansprüche 6 bis 8, **dadurch gekennzeichnet, daß** der Vena-Cava-Filter (17) mit länglichen, beweglichen Verzahnungselementen (171) versehen ist, wobei die Induktivität (25a, 25b) an den Verzahnungselementen (171) befestigt ist, so dass sie sich beim Auffalten der Verzahnungselemente (171) zusammen mit diesen auffaltet.

10. Vena-Cava-Filter nach mindestens einem der Ansprüche 6 bis 9, **dadurch gekennzeichnet, daß** die Induktität (25a, 25b) und/oder Kapazität (35a, 35b) aus dem Material des Vena Cava Filters gebildet sind.

11. Vena-Cava-Filter nach mindestens einem der Ansprüche 6 bis 10, **dadurch gekennzeichnet, daß** der Vena-Cava-Filter Mittel (113) aufweist, welche mindestens einen Schwingkreis bei Einstrahlung der hochfrequenten Strahlung verstimmen.

12. Vena-Cava-Filter nach mindestens einem der Ansprüche 6 bis 11, **dadurch gekennzeichnet, daß** der Vena-Cava-Filter Mittel (112) zum Kurzschließen der Kapazität (3') bei Einstrahlung der hochfrequenten Strahlung aufweist.

13. Vena-Cava-Filter nach mindestens einem der Ansprüche 6 bis 12, **dadurch gekennzeichnet, daß** ein Schalter (10) vorgesehen ist, durch den der mindestens eine Schwingkreis aktivierbar bzw. deaktivierbar ist.

## Revendications

1. Système d'imagerie par résonance magnétique avec :
a) des moyens pour la production d'un champ magnétique extérieur autour d'un objet d'étude,
b) des moyens pour l'irradiation d'un rayonnement haute fréquence d'une fréquence de résonance déterminée dans l'objet d'étude, moyennant quoi des passages entre les niveaux d'énergie-Spin des noyaux d'atome de l'objet d'étude sont engendrés,
c) un dispositif médical pouvant être introduit dans l'objet d'étude avec au moins un circuit oscillant fermé présentant une inductance (22a, 22b, 25a, 25b) et une capacitance (32a, 32b, 35a, 35b), dont la fréquence de résonance est sensiblement identique à la fréquence de résonance du rayonnement haute fréquence irradié, et
d) au moins une bobine de réception pour la détection d'une réponse de signal
**caractérisé en ce que**
une partie repliable du dispositif crée l'inductance (22a, 22b, 25a, 25b) ou **en ce que** l'inductance (22a, 22b, 25a, 25b) est intégrée dans cette partie de façon à ce qu'elle se replie conjointement avec cette dernière lors du repli du dispositif dans l'objet d'étude.

2. Système d'imagerie par résonance magnétique selon la revendication 1, **caractérisé en ce que** le dispositif présente des moyens (113) qui désaccordent au moins un circuit oscillant lors de l'irradiation du rayonnement haute fréquence.

3. Système d'imagerie par résonance magnétique selon la revendication 1 ou 2, **caractérisé en ce que** le dispositif présente des moyens (112) pour court-circuiter la capacitance (3') lors de l'irradiation du rayonnement haute fréquence.

4. Système d'imagerie par résonance magnétique selon au moins l'une des revendications 1 à 3, **caractérisé en ce que** l'inductance (2) et/ou la capacitance (3) du circuit oscillant sont réglables pour s'accorder à la fréquence de résonance du système par résonance magnétique.

5. Système d'imagerie par résonance magnétique selon au moins l'une des revendications 1 à 4, **caractérisé en ce que** le circuit oscillant (4) présente plusieurs inductances (2a, 2n) et/ou capacitances (3a, 3n) commutées en parallèle ou en série.

6. Filtre à veine cave repliable pour une utilisation dans un système d'imagerie par résonance magnétique selon la revendication 1, **caractérisé par**
au moins un circuit oscillant fermé avec une inductance (25a, 25b) et une capacitance (35a, 35b), moyennant quoi l'inductance (25a, 25b) est intégrée dans une partie repliable du filtre à veine cave de façon à ce qu'elle se replie conjointement avec cette dernière lors du repli du filtre à veine cave.

7. Filtre à veine cave selon la revendication 6, **caractérisé en ce que** l'inductance (25a, 25b) est formée en spirale ou en hélice.

8. Filtre à veine cave selon la revendication 6 ou 7, **caractérisé en ce que** la capacitance (35a, 35b) est formée par des éléments capacitifs annulaires parallèles.

9. Filtre à veine cave selon au moins l'une des revendications 6 à 8, **caractérisé en ce que** le filtre à veine cave (17) est doté d'éléments d'engrènement mobiles, longitudinaux (171), moyennant quoi l'inductance (25a, 25b) est fixée aux éléments d'engrènement (171) de façon à ce qu'elle se replie avec ces derniers lors du repli des éléments d'engrènement (171).

10. Filtre à veine cave selon au moins l'une des revendications 6 à 9, **caractérisé en ce que** l'inductance (25a, 25b) et/ou la capacitance (35a, 35b) sont formées à partir du matériau du filtre à veine cave.

11. Filtre à veine cave selon au moins l'une des revendications 6 à 10, **caractérisé en ce que** le filtre à veine cave présente des moyens (113) qui désaccordent au moins un circuit oscillant lors de l'irradiation du rayonnement haute fréquence.

12. Filtre à veine cave selon au moins l'une des revendications 6 à 11, **caractérisé en ce que** le filtre à veine cave présente des moyens (112) pour court-circuiter la capacitance (3') lors de l'irradiation du rayonnement haute fréquence.

13. Filtre à veine cave selon au moins l'une des revendications 6 à 12, **caractérisé en ce qu'**un interrupteur (10) est prévu, grâce auquel au moins un circuit oscillant peut être activé ou désactivé.

## Claims

1. MR-imaging system with
a) means for creating an external magnetic field around an object under investigation;
b) means for beaming high-frequency radiation of a certain resonance frequency into the object under investigation wherein changes between the spin-energy level of the atomic cores of the object under investigation are excited;
c) a medicinal device which can be introduced into the object under investigation and which has at least one closed vibration circuit with an inductance (22a, 22b, 25a, 25b) and a capacitance (32a, 32b, 35a, 35b) whose resonance frequency is substantially equal to the resonance frequency of the radiated high-frequency radiation, and
d) at least one receiver coil for detecting a signal response,
**characterised in that**
an unfolding part of the device forms the inductance (22a, 22b, 25a, 25b) or the inductance (22a, 22b, 25a, 25b) is integrated in one such part so that it unfolds at the same time as the device is unfolded in the object under investigation.

2. MR-imaging system according to claim 1, **characterised in that** the device has means (113) which detunes at least one vibration circuit during irradiation of the high-frequency radiation.

3. MR-imaging system according to claim 1 or 2, **characterised in that** the device has means (112) for short-circuiting the capacitance (3') during irradiation of the high-frequency radiation.

4. MR-imaging system according to at least one of the claims 1 to 13, **characterised in that** the inductance (2) and/or the capacitance (3) of the vibration circuit can be adjusted to match the resonance frequency of the MR-system.

5. MR-imaging system according to at least one of claims 1 to 14, **characterised in that** the vibration circuit (4) has several inductances (2a, 2n) and/or capacitances (3a, 3n) connected in parallel or in series.

6. Unfolding vena-cava filter for use in an MR-imaging system according to claim 1,
**characterised by**
at least one closed vibration circuit with an inductance (25a, 25b) and a capacitance (35a, 35b) wherein the inductance (25a, 25b) is integrated in an unfolding part of the vena-cava filter so that it unfolds at the same time as the vena-cava filter is unfolded.

7. Vena-cava filter according to claim 6 **characterised in that** the inductance (25a, 25b) is shaped in a spiral or helix.

8. Vena-cava filter according to claim 6 or 7, **characterised in that** the capacitance (35a, 35b) is formed by parallel ring-shaped capacitative elements.

9. Vena-cava filter according to at least one of claims 6 to 8, **characterised in that** the vena-cava filter (17) is provided with oblong movable toothed elements (171) wherein the inductance (25a, 25b) is fixed on the toothed elements (171) so that it unfolds at the same time as the toothed elements (171) unfold.

10. Vena cava filter according to at least one of claims 6 to 9, **characterised in that** the inductance (25a, 25b) and/or capacitance (35a, 35b) are formed from the material used for the vena cava filter.

11. Vena cava filter according to at least one of claims 6 to 10, **characterised in that** the vena cava filter has means (113) which detune at least one vibration circuit during irradiation of the high-frequency radiation.

12. Vena cava filter according to at least one of claims 6 to 11, **characterised in that** the vena cava filter has means (112) for short-circuiting the capacitance (3') during irradiation of the high-frequency radiation.

13. Vena cava filter according to at least one of claims 6 to 12, **characterised in that** a switch (10) is provided through which the at least one vibration circuit can be activated and deactivated.
